# EUROPEAN PATENT APPLICATION

(11) **EP 4 299 055 A1**
(43) Date of publication of application: **03.01.2024**
(21) Application number: 23181589.5
(22) Date of filing: 26.06.2023
(51) Int. Cl.: A61K 9/20, A61K 9/28, A61K 31/7048

(54) **ORAL DOSAGE FORMULATIONS COMPRISING EMPAGLIFLOZIN**

(30) Priority: 29.06.2022 TR 202210736
(71) Applicant: SANOVEL ILAC SANAYI VE TICARET A.S., Istanbul 34460 (TR)
(72) Inventor: GULER, Tolga, Istanbul (TR); ARMUT, Merve, Istanbul (TR); PEHLIVAN AKALIN, Nur, Istanbul (TR); SUNEL, Fatih, Istanbul (TR)
(74) Representative: Sevinç, Erkan

(57) **Abstract**

The present invention relates to oral dosage formulations comprising Empagliflozin or a pharmaceutically acceptable salt thereof and at least one filler wherein Empagliflozin or pharmaceutically acceptable salt has a d (0.9) particle size higher than 150 µm.

## Description

### Field of the Invention

The present invention relates to oral dosage formulations comprising Empagliflozin or a pharmaceutically acceptable salt thereof and at least one filler wherein Empagliflozin or pharmaceutically acceptable salt has a d (0.9) particle size higher than 150 µm.

### Background of the Invention

Empagliflozin is a potent inhibitor of sodium-glucose co-transporter type 2 (SGLT-2) and is therefore used to treat type 2 diabetes. It is currently approved for the treatment of type 2 diabetes and improvement of blood sugar control.

Empagliflozin is a white to yellowish non-hygroscopic crystalline solid, very slightly soluble in water (pH 1-7.4), slightly soluble in acetonitrile and ethanol, sparingly soluble in methanol, and practically insoluble in toluene. The chemical name of empagliflozin (1S)-1,5-anhydro-1-(4-chloro-3-{4-[(3S)-tetrahydrofuran-3-yloxy]benzyl}phenyl)-D- glucitol and its chemical structure is shown in the Formula- I.

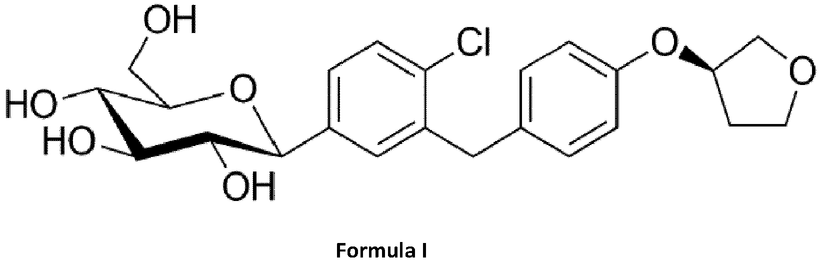

Empagliflozin is available on the market in the form of a free base and is sold under trade name Jardiance<^{®}>, as an oral tablet in 10 mg and 25 mg strengths. Further it is available on the market as a combination product with Metformin and a combination product with Linagliptin.

The first mention of the crystalline form of empagliflozin can be found in patent application WO 2006/117359. WO2006117360 discloses two other forms of empagliflozin which is described as Form I and Form II. Also, it disclosed methods for production thereof and synthesis of Empagliflozin.

As evident from the prior art and the literature there are reported several pharmaceutical compositions comprising Empagliflozin. There is an existing and continual need for oral dosage formulations comprising Empagliflozin or a pharmaceutically acceptable salt thereof that have good content uniformity, improved compressibility and manufactured by safe, effective, easy manufacturing methods.

### Detailed Description of the Invention

The main object of the present invention is to provide oral dosage formulations comprising a therapeutically effective amount of Empagliflozin or a pharmaceutically acceptable salt thereof with good content uniformity.

Another object of the present invention is to provide oral dosage formulations comprising Empagliflozin or a pharmaceutically acceptable salt thereof with improved compressibility, flowability and high stability.

Another object of the present invention is to provide oral dosage formulations comprising Empagliflozin or a pharmaceutically acceptable salt thereof prepared by simple, easy, time-saving and fast manufacturing methods.

According to one embodiment of this invention, oral dosage formulations comprising Empagliflozin or a pharmaceutically acceptable salt thereof and at least one filler wherein Empagliflozin or a pharmaceutically acceptable salt has a d (0.9) particle size higher than 150 µm.

Small particles can be important for oral dosage formulations. In the state of the art, small particle desirable for the formulations because of affecting the dissolution properties or bioavailability. However, small particles can also be negatively affect the formulations.

We have found that Empagliflozin with a small particle size presents problems and pharmaceutical compositions comprising it show manufacturability problem for example by sticking and small particles negatively affect the content uniformity of the pharmaceutical composition.

Even more surprisingly, we have found that oral dosage compositions comprising Empagliflozin or a pharmaceutically acceptable salt thereof wherein the particle size is d (0.9) higher than 150 µm may exhibit excellent content uniformity and high bioavailability. In the following preferred range of the particle size distribution are described. This choice of particle size eliminated the described above problems.

The term "Dx" as used herein means that x% of the particles in a composition (based on volume) have a diameter of or below a specified d value. D (0.9)" or "d90" means that the size at which %90 by volume of the particles are finer. The volume mean particle size of the compound of the Empagliflozin is determined using Malvern Mastersizer 2000 laser diffraction particle size analyzer.

According to one embodiment of this invention, Empagliflozin or a pharmaceutically acceptable salt thereof has a d (0.9) particle size higher than 150 µm. These particle size helps to provide the desired content uniformity.

According to one embodiment, Empagliflozin or a pharmaceutically acceptable salt thereof has a d (0.9) particle size smaller than 250 µm.

According to this embodiment, Empagliflozin or a pharmaceutically acceptable salt thereof has a d (0.9) particle size between 150 µm to 180 µm, between 180 µm to 200 µm, between 200 µm to 210 µm, between 210 µm to 230 µm, between 230 µm to 250 µm.

According to one embodiment, Empagliflozin or a pharmaceutically acceptable salt thereof has a d (0.9) particle size smaller than 350 µm.

According to this embodiment, Empagliflozin or a pharmaceutically acceptable salt thereof has a d (0.9) particle size between 250 µm to 270 µm, between 270 µm to 300 µm, between 300 µm to 320 µm, between 320 µm to 350 µm.

According to one embodiment of the present invention, the amount of Empagliflozin is present between 0.5% and %25.0 by weight in the total composition. Preferably, the amount of Empagliflozin is between 10.0% and 15.0% or 15.0% and 15.0% by weight in the total composition.

Suitable fillers are selected from the group comprising lactose, lactose monohydrate, spray-dried lactose monohydrate, anhydrous lactose, microcrystalline cellulose, pregelatinized starch, mannitol, corn starch, maltodextrin, dextrin, dextrose, erytritol, fructose, maltose, sucrose, xylitol, dicalcium phosphate, hydroxypropyl betadex, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, medium chain triglycerides, polydextrose, polymethacrylates, sodium alginate, sodium chloride, sorbitol, starch, sugar sphericals, sulfobutylether beta-cyclodextrin, tragacanth, trehalose, polysorbate 80 or mixtures thereof.

According to an embodiment of the present invention, the amount of fillers are 5.0% to 95.0% by weight in the total composition. Preferably, it is between 35.0% to 90.0% or between 50.0% and 90.0% by weight in the total formulation.

According to one embodiment of the present invention, the filler is microcrystalline cellulose.

According to one embodiment of the present invention, the filler is anhydrous lactose.

According to one embodiment of the present invention, the fillers are microcrystalline cellulose and anhydrous lactose.

According to an embodiment of the present invention, the oral dosage compositions further comprise at least one glidants/lubricants.

Suitable glidants/lubricants are selected from the group comprising anhydrous colloidal silicon dioxide, magnesium stearate, sodium stearyl fumarate, magnesium oxide, starch, silicone dioxide, talc, polyethylene glycol, stearic acid, aluminum silicate, magnesium silicate, colloidal silica, or mixtures thereof.

According to an embodiment of the present invention, the glidant/lubricant is anhydrous colloidal silicon dioxide or magnesium stearate or a mixture thereof. These excipients provide the flowability of the mixture.

In one embodiment, the oral dosage composition is in the form of capsule or tablet or film coated tablet.

The oral dosage formulation of the present invention is a tablet, especially a film coated tablet and is prepared by direct compression.

According to an embodiment of the present invention, the oral dosage formulation comprises;
a) Empagliflozin
b) Microcrystalline Cellulose
c) Anhydrous Lactose
d) Anhydrous colloidal silicon dioxide
e) Magnesium stearate
f) Coating material.

According to this embodiment, the coating material can include one or more following excipients of hydroxypropyl methylcellulose, hydroxypropyl cellulose, polyvinyl alcohol (PVA), polyethylene glycol (PEG), glycerin, talc, polyvinyl alcohol-polyethylene glycol copolymers, ^{®}), polyvinylprolidone, polyvinylprolidone-vinyl acetate copolymer (PVP-VA), iron oxide yellow, iron oxides, all kinds of Opadry^{®}, pigments, dyes, titanium dioxide, coloring agent or mixtures thereof.

According to one embodiment of the present invention, a process for the preparation of the oral dosage formulation comprises;
a) Mixing Empagliflozin, microcrystalline cellulose, anhydrous lactose and anhydrous colloidal silicon dioxide,
b) Mixing and sieving the mixture,
c) Adding magnesium stearate and then mixing,
d) Compressing the mixture into the tablet,
e) Coating the tablets with film coating agent.

### Example 1: Film coated tablet comprising empagliflozin

| **Ingredients** | **Amount (% by weight of the total formulation)** |
|---|---|
| Empagliflozin | 0.5-25 |
| Microcrystalline Cellulose | 5-70 |
| Anhydrous lactose | 5-70 |
| Anhydrous colloidal silicon dioxide | 0.1-10 |
| Magnesium stearate | 0.1-10 |
| Coating | 0.5-4.0 |
| **TOTAL** | **100** |

## Claims

1. An oral dosage formulation comprising Empagliflozin or pharmaceutically acceptable salt thereof and at least one filler wherein Empagliflozin or pharmaceutically acceptable salt has a d (0.9) particle size higher than 150 µm.

2. The oral dosage formulation according to claim 1, wherein Empagliflozin has a d (0.9) particle size smaller than 250 µm.

3. The oral dosage formulation according to claim 1, wherein Empagliflozin has a d (0.9) particle size smaller than 350 µm.

4. The oral dosage formulation according to claim 1, wherein fillers are selected from the group comprising lactose, lactose monohydrate, spray-dried lactose monohydrate, anhydrous lactose, microcrystalline cellulose, pregelatinized starch, mannitol, corn starch, maltodextrin, dextrin, dextrose, erytritol, fructose, maltose, sucrose, xylitol, dicalcium phosphate, hydroxypropyl betadex, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, medium chain triglycerides, polydextrose, polymethacrylates, sodium alginate, sodium chloride, sorbitol, starch, sugar sphericals, sulfobutylether beta-cyclodextrin, tragacanth, trehalose, polysorbate 80 or mixtures thereof.

5. The oral dosage formulation according to claim 4, wherein the fillers are microcrystalline cellulose or anhydrous lactose or mixtures thereof.

6. The oral dosage formulation according to claim 5, wherein the amount of filler is between 2.0% to 85, between 5.0% to 70% by weight of the total composition.

7. The oral dosage formulation according to claim 1, further comprising at least one glidant/lubricant.

8. The oral dosage formulation according to claim 7, wherein the glidant/lubricants are selected from the group comprising anhydrous colloidal silicon dioxide, magnesium stearate, sodium stearyl fumarate, magnesium oxide, starch, silicone dioxide, talc, polyethylene glycol, stearic acid, aluminum silicate, magnesium silicate, colloidal silica or mixtures thereof.

9. The oral dosage form according to any preceding claims, wherein the dosage form is in the form of capsule or tablet or film coated tablet.

10. The oral dosage formulation according to claim 9, wherein the dosage form is film coated tablet and the film coated tablet is obtained by direct compression.

11. The oral dosage formulation according to any preceding claims, comprising;
a) Empagliflozin has a d (0.9) particle size higher than 150 µm,
b) Microcrystalline cellulose,
c) Lactose, anhydrous,
d) Anhydrous Colloidal Silicon dioxide,
e) Magnesium stearate.

12. A process for preparation of the oral dosage formulation according to any preceding claims comprising the following steps of
a) Mixing Empagliflozin, microcrystalline cellulose, anhydrous lactose and anhydrous colloidal silicon dioxide,
b) Mixing and sieving the mixture,
c) Adding magnesium stearate and then mixing,
d) Compressing the mixture into the tablet,
e) Coating the tablets with film coating agent.
